# DEMANDE DE BREVET EUROPEEN

(11) **EP 1 163 899 A1**
(43) Date de publication de la demande: **19.12.2001**
(21) Numéro de dépôt: 01401504.4
(22) Date de dépôt: 11.06.2001
(51) Int. Cl.: A61K 7/043, A61K 7/032, A61K 7/025

(54) **Composition cosmétique filmogène**

(30) Priorité: 15.06.2000 FR 0007655
(71) Demandeur: L'OREAL, 75008 Paris (FR)
(72) Inventeur: De La Poterie, Valérie, 77820 Le Châtelet-en-Brie (FR)
(74) Mandataire: Kromer, Christophe

(57) **Abrégé**

L'invention concerne une composition cosmétique comprenant une dispersion aqueuse de particules de polymère filmogène radicalaire et une dispersion aqueuse d'un polymère ayant au moins une chaîne grasse, en particulier d'une résine alkyde, ayant une longueur en huile allant de 5 % à 90 %, et
- lorsque le rapport pondéral polymère radicalaire/résine alkyde est inférieur à 1,5, la résine alkyde a une longueur en huile inférieure à 50 % ;
- lorsque le rapport pondéral polymère radicalaire/résine alkyde est supérieur ou égal à 1,5, le polymère acrylique a une température de transition vitreuse (Tg) inférieure ou égale à 30 °C.

Application au maquillage et au traitement des matières kératiniques.

## Description

La présente invention a pour objet une composition cosmétique filmogène comprenant une dispersion aqueuse de polymère de type radicalaire filmogène et un polymère à chaîne grasse utilisable notamment comme composition de maquillage ou de soin des matières kératiniques telles que la peau, y compris les lèvres, les ongles, les cils, les sourcils, les cheveux d'êtres humains. L'invention se rapporte aussi à une utilisation de cette composition pour le traitement et le soin des matières kératiniques. Elle est destinée plus spécialement au traitement et au soin des ongles.

De façon plus précise, l'invention se rapporte à une composition contenant des polymères filmogènes, capable de former sur un support (ongle, cil, cheveu) un film homogène et continu.

Parmi les principales caractéristiques que doivent posséder les compositions cosmétiques filmogènes telles que les vernis à ongles, on peut citer l'obtention d'un film homogène, ayant une certaine flexibilité et une bonne résistance du film, par exemple en vue d'éviter les craquelures et l'écaillement du film de polymère.

Les compositions cosmétiques filmogènes comprenant des polymères filmogènes en dispersion aqueuse sont actuellement en plein développement, notamment comme produit de maquillage, en vue de se substituer aux compositions en milieu solvant, comme les vernis-à-ongles solvants, non seulement pour des raisons de sécurité pour la consommatrice, mais aussi pour des raisons d'environnement du fait de l'absence de composés volatils dans de telles compositions.

Ces compositions cosmétiques filmogènes en milieu aqueux comprennent soit un polymère filmogène filmifiant à température ambiante mais certains polymères conduisent à la formation de film mou, voire collant, ne permettant pas l'obtention d'un film résistant; soit un polymère filmogène ayant une température de transition élevée (supérieure à la température ambiante, soit environ 25 °C) et il est alors nécessaire d'associer à ce polymère un agent plastifiant et/ou un agent de coalescence afin d'obtenir un film à température ambiante présentant des propriétés satisfaisantes. Ainsi, le brevet US-A-4158053 décrit l'emploi d'éthers de glycol comme agent de coalescence dans des compositions de vernis-à-ongles comprenant des polymères acryliques en dispersion aqueuse. Toutefois, ces éthers de glycol présentent des risques toxicologiques limitant, voire interdisant, leur emploi dans ces compositions. De même, la nécessité de limiter l'emploi de cosolvants dans le but de préparer des produits sans composés organiques volatils (dits produits non VOC) ne permet pas d'obtenir des films ayant les propriétés optimales recherchées. Un besoin existe donc de disposer d'autres composés, autres que des cosolvants, pouvant agir comme plastifiant du film.

Le but de la présente invention est de proposer une composition filmogène, bien adaptée au maquillage et au soin des matières kératiniques, présentant de bonnes propriétés cosmétiques et ne présentant pas les inconvénients mentionnés ci-dessus.

Le demandeur a découvert qu'une telle composition pouvait être obtenue en utilisant une dispersion aqueuse de polymère filmogène radicalaire et un polymère ayant au moins une chaîne grasse. L'association de ces polymères selon l'invention conduit à la formation d'un film suffisamment résistant pour éviter l'apparition de craquelures, sans utiliser de cosolvant.

Le document EP-A-418469 décrit un vernis à ongles comprenant une dispersion aqueuse de polymère filmogène et une résine alkyde.

De façon plus précise, l'invention a donc pour objet une composition cosmétique comprenant une dispersion aqueuse de particules de polymère de type radicalaire filmogène et une dispersion aqueuse d'un polymère ayant au moins une chaîne grasse, caractérisée par le fait que le polymère ayant au moins une chaîne grasse a une longueur en huile allant de 5 % à 90 %, et
- lorsque le rapport pondéral polymère radicalaire /polymère à chaîne grasse est inférieur à 1,5, le polymère à chaîne grasse a une longueur en huile inférieure à 50 % ;
- lorsque le rapport pondéral polymère radicalaire /polymère à chaîne grasse est supérieur ou égal à 1,5, le polymère acrylique a une température de transition vitreuse (Tg) inférieure ou égale à 30 °C.

L'invention a encore pour objet un vernis-à-ongles et/ou un produit de soin des ongles consistant en une composition telle que définie précédemment.

L'invention a aussi pour objet un procédé de traitement cosmétique ou de maquillage des matières kératiniques caractérisé par le fait que l'on applique sur les matières kératiniques une composition telle que définie précédemment.

L'invention a également pour objet l'utilisation d'une dispersion aqueuse de polymère de type radicalaire filmogène et d'une dispersion aqueuse d'un polymère ayant au moins une chaîne grasse tels que définis précédemment pour l'obtention d'un film. L'invention a en outre pour objet l'utilisation d'une dispersion aqueuse d'un polymère ayant au moins une chaîne grasse dans une composition telle que définie précédemment comme agent plastifiant du polymère de type radicalaire filmogène.

La composition cosmétique selon l'invention comprend un milieu cosmétiquement acceptable, c'est-à-dire un milieu compatible avec les matières kératiniques.

Le polymère ayant au moins une chaîne grasse utilisé selon l'invention peut être un polycondensat, en particulier un polycondensat choisi parmi les polyesters, les polyesters-amides, les polyuréthanes et les polyurées. Avantageusement, le polymère ayant au moins une chaîne grasse est une résine alkyde. On entend par "chaîne grasse" une chaîne hydrocarbonée comprenant au moins 8 atomes de carbone, notamment de 8 à 46 atomes de carbone ; on entend par longueur en huile d'un polymère ayant au moins une chaîne grasse, le pourcentage pondéral en chaîne grasse dans ledit polymère.

Les résines alkydes utilisées selon l'invention sont des polyesters comprenant des chaînes hydrocarbonées d'acides gras. De telles résines sont notamment décrites dans l'Encyclopedia of chemical technology - Kirk-Othmer, 4^{ème} édition, volume 2, pages 53 à 63 dont le contenu est incorporé à titre de référence dans la présente demande. Ces résines sont obtenues par polymérisation de polyols et de polyacides ou de leur anhydride correspondant, en présence d'acides gras. Ces acides gras peuvent être employés tels quels ou bien sous forme de triglycérides d'acides gras ou bien encore présents dans les huiles lors de la synthèse de la résine alkyde.

Du fait de la présence de chaînes hydrocarbonées d'acides gras dans la résine alkyde, on défini couramment les résines alkyde par leur longueur en huile. Ainsi, on entend selon la présente demande par longueur en huile d'une résine alkyde, le pourcentage pondéral en chaîne hydrocarbonées d'acide gras présent dans la résine alkyde.

Comme polyols pouvant être employés pour la synthèse de résine alkyde, on peut citer le glycérol, le pentaérythritol, le triméthylol propane, le triméthylol éthane, le néopentyl glycol, le propylène glycol, l'éthylène glycol, l'hexane diol-1,6, le butanediol-1,4, le diéthylène glycol.

Comme polyacide ou anhydride, on peut notamment citer l'anhydride phtalique, l'acide isophtalique, l'acide téréphtalique, l'anhydride trimellitique, l'anhydride maléique, l'acide adipique, l'acide fumarique, l'acide azélaïque, l'acide sébacique.
Les acides gras répondent de préférence à la formule R-COOH, dans laquelle R désigne un radical hydrocarboné, saturé ou insaturé, ayant de 7 à 45 atomes de carbone environ, de préférence de 9 à 35 atomes de carbone, avantageusement de 15 à 35 atomes de carbone et mieux de 15 à 21 atomes de carbone.

Comme acide gras, on peut notamment citer l'acide palmitique, l'acide stéarique, l'acide oléique, l'acide ricinoléique, l'acide linoléique, l'acide linolénique.
Les acides gras sont présents dans la majorité des huiles d'origine naturelle, notamment sous la forme de triglycérides. Les triglycérides d'acides gras sont des esters résultant de la réaction des trois fonctions alcools de la glycérine sur des acides gras, ces acides gras pouvant être identiques ou différents. Les huiles d'origine naturelle peuvent donc être utilisées pendant la polymérisation. Elles peuvent être choisies parmi l'huile de lin, l'huile de bois de chine, l'huile de oititica, l'huile de soja, l'huile de tournesol, l'huile de carthame, l'huile de ricin, l'huile de coco (coprah), l'huile d'olive, l'huile de palme, l'huile de colza, l'huile d'arachide, le tallol (dite aussi "tall oil").

Le polymère à chaîne grasse, en particulier la résine alkyde, présent dans la composition selon l'invention est présent sous la forme de dispersion aqueuse et est donc insoluble dans l'eau (sa solubilté dans l'eau à 25 °C étant inférieure à 1 % en poids).

Comme résines alkyde utilisables selon l'invention, on peut notamment citer celles vendues sous les dénominations "NECOWEL 581® " (50 % en huile de soja), "NECOWEL 585® " (20 % en huile de tournesol), "NECOWEL 580® " (20 % en huile), "NECOWEL 586 N® " (50 % en huile de soja), "NECOWEL EP 1161® " (50 % en huile de soja), "NECOWEL EP 1213® " (20 % en huile), "NECOWEL EP 2009® " (32 % en huile de tournesol), "NECOWEL EP 2019® " (20 % en huile), "NECOWEL EP 2275® " (35 % en huile), "NECOWEL EP 2329® " (34 % en huile), "NECOWEL EP 3016® " (30 % en huile) par la société ASHLAND, "URADIL XP 515 AZ® " (73 % en tallol), "URADIL XP 516 AZ® " (63 % en tallol) par la société DSM RESINS.

Dans la composition selon l'invention, le polymère à chaîne grasse peut être présent en une teneur allant de 0,5 % à 40 % en poids, par rapport au poids total de la composition, de préférence de 2 % à 30% en poids, et mieux de 5 % à 20 % en poids.

Les polymères de type radicalaires utilisés selon l'invention peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces monomères acides.

On utilise de préférence des polymères radicalaires anioniques, c'est-à-dire des monomères ayant au moins un monomère à groupement acide, notamment acide carboxylique.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C1-C20, de préférence en C1-C8, des (méth)acrylates d'aryle, en particulier d'aryle en C6-C10, des (méth)acrylates d'hydroxyalkyle, en particulier d'hydroxyalkyle en C2-C6 .
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide et le N-t-octyl acrylamide.

Les polymères vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée ou fluorée).

La taille des particules de polymère en dispersion aqueuse peut aller de 10 à 500 nm, et va de préférence de 20 à 300 nm.

Comme polymère acrylique utilisable selon l'invention, on peut citer ceux vendus sous les dénominations NEOCRYL XK-90® , NEOCRYL A-1070® , NEOCRYL BT-62® , NEOCRYL A-1079® , NEOCRYL A-523® , NEOCRYL XK-52® , NEOCRYL A-639® , par la société ZENECA, DOW LATEX 432® par la société DOW CHEMICAL, CARBOSET CR 712® , CARBOSET CR 714® par la société GOODRICH.

Dans la composition selon l'invention, le polymère radicalaire peut être présent en une teneur allant de 3 % à 50 % en poids, par rapport au poids total de la composition, et de préférence de 10 % à 35 % en poids.

Selon l'invention, lorsque le rapport pondéral polymère filmogène radicalaire/polymère à chaîne grasse est inférieur à 1,5, le polymère à chaîne grasse a de préférence une longueur en huile inférieure à 35 %. Par ailleurs, lorsque le rapport pondéral polymère filmogène radicalaire/polymère à chaîne grasse est supérieur ou égal à 1,5, le polymère radicalaire a une température de transition vitreuse (Tg) allant de - 40 °C à 30 °C ; de préférence, la Tg est inférieure ou égale à 25 °C, et peut aller notamment de
-10 °C à 25 °C.
La mesure de la température de transition vitreuse est effectuée par DSC (Differential Scaning Calorimetry) selon la norme ASTM D3418-97.

En outre, la composition selon l'invention peut contenir des adjuvants couramment utilisés dans les compositions cosmétiques. On peut citer à titre d'exemple d'adjuvants les colorants, les pigments, les nacres, les laques, les agents anti-UV, les agents épaississants, les tensioactifs, les cires, les silicones, les parfums, les conservateurs, les agents hydratants et des actifs de tels que le D-panthénol, le phytantriol, les vitamines, la biotine, les oligo-éléments. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

La composition selon l'invention peut être utilisée pour le maquillage ou le traitement cosmétique des matières kératiniques. La composition de maquillage peut être un vernis à ongles, une composition de soin des ongles, un eye-liner, un mascara, un fond de teint, un fard à paupières ou a joue, un rouge à lèvres, un produit anti-cernes, ou encore un produit de maquillage du corps du type tatouage provisoire ou semi-permanent. La composition de traitement cosmétique peut être une composition de soin pour le visage, le cou, les mains ou pour le corps ; elle peut constituer aussi une composition solaire ou autobronzante.

On va maintenant donner des exemples illustrant la présente invention sans toutefois la limiter.

### Exemple 1 :

On a préparé un eye-liner ayant la composition suivante :
- Dispersion aqueuse de polymère acrylique à 46, 6 %
   de matière active (NEOCRYL A 639 de la société ZENECA) 16,1 g
- Emulsion aqueuse de résine alkyde à 20 % en huile
   et à 44 % dans l'eau (NECOWEL 585 de la société ASHLAND) 39,8 g
- Oxyde de fer noir 15 g
- Propylène glycol 15 g
- Eau qsp 100 g

La composition après application sur le bord des paupières forme un film lisse et homogène.

### Exemple 2 :

On a préparé un produit pour les lèvres ayant la composition suivante :
- Dispersion aqueuse de polymère acrylique à 41 % dans l'eau
   (CARBOSET CR 712 de la société GOODRICH) 34,1 g
- Emulsion aqueuse de résine alkyde à ,50 % en huile
   et à 47 % dans l'eau (NECOWEL 581 de la société ASHLAND) 12,8 g
- Pigments 4 g
- Glycérine 2,5 g
- Eau qsp 100 g

La composition s'applique facilement sur les lèvres et forme sur celles-ci un film lisse et brillant.

### Exemple 3 :

On a préparé un vernis à ongles ayant la composition suivante :
- Dispersionaqueuse de polymère acrylique à 46,6 %
   de matière active (NEOCRYL A 639 de la société ZENECA) 32,2 g
- Dispersion aqueuse de résine alkyde à 20 % en huile et
   à 44 % dans l'eau (NECOWEL 585 de la société ASHLAND) 34,1g
- Pigments 3 g
- Epaississant 0,3 g
- Glycérine 1,9 g
- Eau qsp 100 g

Le vernis s'applique facilement sur l'ongle et laisse après séchage un film résistant et homogène.

### Exemples 4 à 31 comparatifs :

On a préparé des mélanges de dispersion aqueuse de polymère acrylique et d'émulsion de résine alkyde et on a observé si le mélange formait un film.

On a obtenu les résultats suivants :

| **Proportion acrylique/alkyde** | **Résine alkyde** | **Polymère acrylique** | **Exemple** | **Filmification** |
|---|---|---|---|---|
| 50/50 | Necowel 585 | Carboset CR 712 | 4 | Oui |
| | | Carboset CR 714 | 5 | Oui |
| | | Neocryl A 639 | 6 | Oui |
| | | Neocryl XK 52 | 7 | Oui |
| | Necowel 581 | Carboset CR 712 | 8 | Non |
| | | Carboset CR 714 | 9 | Non |
| | | Neocryl A 639 | 10 | Non |
| | | Neocryl XK 52 | 11 | Non |
| | Uradil XP 515 AZ | Carboset CR 712 | 12 | Non |
| | | Carboset CR 714 | 13 | Non |
| | | Neocryl A 639 | 14 | Non |
| | | Neocryl XK 52 | 15 | Non |
| 70/30 | Necowel 585 | Carboset CR 712 | 16 | Oui |
| | | Carboset CR 714 | 17 | Non |
| | | Neocryl A 639 | 18 | Non |
| | | Neocryl XK 52 | 19 | Non |
| | Necowel 581 | Carboset CR 712 | 20 | Oui |
| | | Carboset CR 714 | 21 | Non |
| | | Neocryl A 639 | 22 | Non |
| | | Neocryl XK 52 | 23 | Non |
| | Uradil XP 515 AZ | Carboset CR 712 | 24 | Oui |
| | | Carboset CR 714 | 25 | Non |
| | | Neocryl A 639 | 26 | Non |
| | | Neocryl XK 52 | 27 | Non |
| 30/70 | Necowel 585 | Carboset CR 712 | 28 | Oui |
| | | Carboset CR 714 | 29 | Oui |
| | | Neocryl A 639 | 30 | Oui |
| | | Neocryl XK 52 | 31 | Oui |

La proportion acrylique/alkyde correspond au rapport pondéral en matière active polymère filmogène radicalaire / résine alkyde.

Le poids total de polymère acrylique et de résine alkyde est de 28 g pour 100 g de composition, le complément étant de l'eau.

Necowel 585 : résine alkyde à 20 % d'huile de tournesol vendue par la société ASLAND

Necowel 581 : résine alkyde à 50 % d'huile de soja vendue par la société ASHLAND

Uradil XP 515 AZ : résine alkyde à 73 % d'huile de suif vendue par la société DSM RESINS

Carboset CR 712 : copolymère acrylique-styrène en émulsion aqueuse à 40 % vendu par la société GOODRICH (Tg = 18 °C)

Carboset CR 714 :copolymère styrène-acrylique en émulsion aqueuse à 42,5 % vendu par la société GOODRICH (Tg = 35 °C)

Neocryl A 639 : copolymère styrène-acrylique en émulsion aqueuse à 45 % vendu par la société ZENECA (Tg = 67 °C)

Neocryl XK 52 : copolymère acrylique en émulsion aqueuse à 40 % vendu par la société ZENECA (Température de transition vitreuse (Tg >100 °C)

On constate que lorsque le rapport pondéral polymère filmogène acrylique/résine alkyde est supérieur à 1,5 (rapport acrylique/alkyde = 70/30), on obtient un film que lorsque le polymère acrylique à une température de transition vitreuse inférieure ou égale à 30 °C (exemples 16, 20 et 24).

Lorsque le rapport pondéral polymère filmogène acrylique/résine alkyde est inférieur à 1,5 (rapport acrylique/alkyde = 50/50 ou 30/70), on obtient un film lorsque la longueur en huile de la résine alkyde est inférieure à 50 % (exemples 4 à 7).

## Revendications

1. Composition cosmétique comprenant une dispersion aqueuse de particules de polymère filmogène radicalaire et une dispersion aqueuse de polymère ayant au moins une chaîne grasse, **caractérisée par le fait que** le polymère à chaîne grasse a une longueur en huile allant de 5 % à 90 %, et
- lorsque le rapport pondéral polymère radicalaire/polymère à chaîne grasse est inférieur à 1,5, le polymère à chaîne grasse a une longueur en huile inférieure à 50 % ;
- lorsque le rapport pondéral polymère radicalaire/polymère à chaîne grasse est supérieur ou égal à 1,5, le polymère acrylique a une température de transition vitreuse (Tg) inférieure ou égale à 30 °C.

2. Composition cosmétique selon la revendication 1, **caractérisée par le fait que** lorsque le rapport pondéral polymère radicalaire/polymère à chaîne grasse est inférieur à 1,5, le polymère à chaîne grasse a une longueur en huile inférieure à 35 %.

3. Composition cosmétique selon la revendication 1, **caractérisée par le fait que** lorsque le rapport pondéral polymère radicalaire/polymère à chaîne grasse est supérieur ou égal à 1,5, le polymère radicalaire a une température de transition vitreuse (Tg) inférieure ou égale à 25 °C.

4. Composition selon l'une quelconque des revendications précédentes, **caractérisée par le fait que** le polymère ayant au moins une chaîne grasse est une résine alkyde.

5. Composition cosmétique selon l'une quelconque des revendications précédentes, **caractérisée par le fait que** le polymère radicalaire est présent en une teneur allant de 3 % à 50 % en poids, par rapport au poids total de la composition.

6. Composition cosmétique selon l'une quelconque des revendications précédentes, **caractérisée par le fait que** le polymère de type radicalaire est un polymère vinylique.

7. Composition cosmétique selon l'une quelconque des revendications précédentes, **caractérisée par le fait que** le polymère de type radicalaire est un polymère acrylique.

8. Composition cosmétique selon l'une quelconque des revendications précédentes, **caractérisée par le fait que** le polymère de type radicalaire résulte de la polymérisation de monomères à insaturation éthylénique ayant au moins un groupement acide et/ou des esters de ces monomères acides et/ou des amides de ces monomères acides.

9. Composition selon l'une quelconque des revendications précédentes, **caractérisée par le fait que** le polymère ayant au moins une chaîne grasse est présent en une teneur allant de 0,5 % à 40 % en poids, par rapport au poids total de la composition, et mieux de 2 % à 30 % en poids.

10. Composition selon l'une quelconque des revendications précédentes, **caractérisée par le fait qu'**elle comprend en outre un adjuvant choisi dans le groupe formé par les colorants, les pigments, les nacres, les laques, les agents anti-UV, les agents épaississants, les tensioactifs, les cires, les silicones, les parfums, les conservateurs, les agents hydratants et les actifs.

11. Composition selon l'une quelconque des revendications précédentes, **caractérisée par le fait qu'**elle se présente sous la forme d'un vernis à ongles, d'une composition de soin des ongles, d'un eye-liner, d'un mascara, d'un fond de teint, d'un fard à paupières ou a joue, d'un rouge à lèvres, d'un produit anti-cernesd'un produit de maquillage du corps du type tatouage provisoire ou semi-permanent, d'une composition de soin pour le visage, le cou, les mains ou pour le corps, d'une composition solaire ou autobronzante.

12. Procédé de traitement cosmétique ou de maquillage des matières kératiniques **caractérisé par le fait que** l'on applique sur les matières kératiniques une composition selon l'une quelconque des revendications 1 à 11.

13. Utilisation d'une dispersion aqueuse de polymère de type radicalaire filmogène et d'une dispersion aqueuse de polymère ayant au moins une chaîne grasse dans une composition cosmétique telle que définie selon l'une quelconque des revendications 1 à 11 pour l'obtention d'un film.

14. Utilisation selon la revendication 13, **caractérisée par le fait que** le polymère ayant au moins une chaîne grasse est une résine alkyde.

15. Utilisation d'une dispersion aqueuse d'un polymère ayant au moins une chaîne grasse dans une composition cosmétique comprenant au moins une dispersion aqueuse de polymère radicalaire filmogène selon l'une quelconque des revendications 1 à 11 comme agent plastifiant dudit polymère radicalaire filmogène.

16. Utilisation selon la revendication 15, **caractérisée par le fait que** le polymère ayant au moins une chaîne grasse est une résine alkyde.
